(19) Europäisches Patentamt
European Patent Office
Office européen des brevets

(11) EP 0 711 837 A2

(12) EUROPÄISCHE PATENTANMELDUNG

(43) Veröffentlichungstag:
**15.05.1996 Patentblatt 1996/20**

(51) Int Cl.6: **C12Q 1/00**, C12M 1/40
// C12Q1/54

(21) Anmeldenummer: **96101100.4**

(22) Anmeldetag: **13.12.1993**

(84) Benannte Vertragsstaaten:
**DE GB**

(30) Priorität: **15.12.1992 AT 2485/92**

(62) Anmeldenummer der früheren Anmeldung nach Art. 76 EPÜ: **93890240.0**

(71) Anmelder: **AVL Medical Instruments AG**
**CH-8201 Schaffhausen (CH)**

(72) Erfinder:
- **Offenbacher, Helmut, Dr.**
**A-8020 Graz (AT)**
- **Marsoner, Hermann, Dipl.-Ing. Dr.**
**A-8151 Steinberg (AT)**

(74) Vertreter: **Krause, Walter, Dr. Dipl.-Ing. et al**
**Postfach 200**
**Singerstrasse 8**
**A-1014 Wien (AT)**

Bemerkungen:
Diese Anmeldung ist am 26 - 01 - 1996 als Teilanmeldung zu der unter INID-Kode 62 erwähnten Anmeldung eingereicht worden.

(54) **Amperometrische Enzymelektrode**

(57) Eine amperometrische Enzymelektrode zur Messung der Konzentration eines Enzymsubstrates in einer Probe weist ein elektrisch leitendes, poröses Elektrodenmaterial (4) mit einem darauf bzw. darin immobilisierten bzw. adsorbierten Enzym (13) auf. Das Elektrodenmaterial (4) besteht aus einem redoxinaktiven Leiter mit einem leitfähigen Pigment (8'), einem selbst nicht leitenden Bindemittel (8) und einer darin fein verteilten katalytischen Substanz (9). Zur Unterdrückung von Interferenzen redoxaktiver Metabolite weist das Elektrodenmaterial (4) der Enzymelektrode eine probenseitige Deckschicht (5) oder eine in den Poren des Elektrodenmaterials (4) vorliegende Sperrschicht auf, welche aus einem polyionischen Polymer besteht.

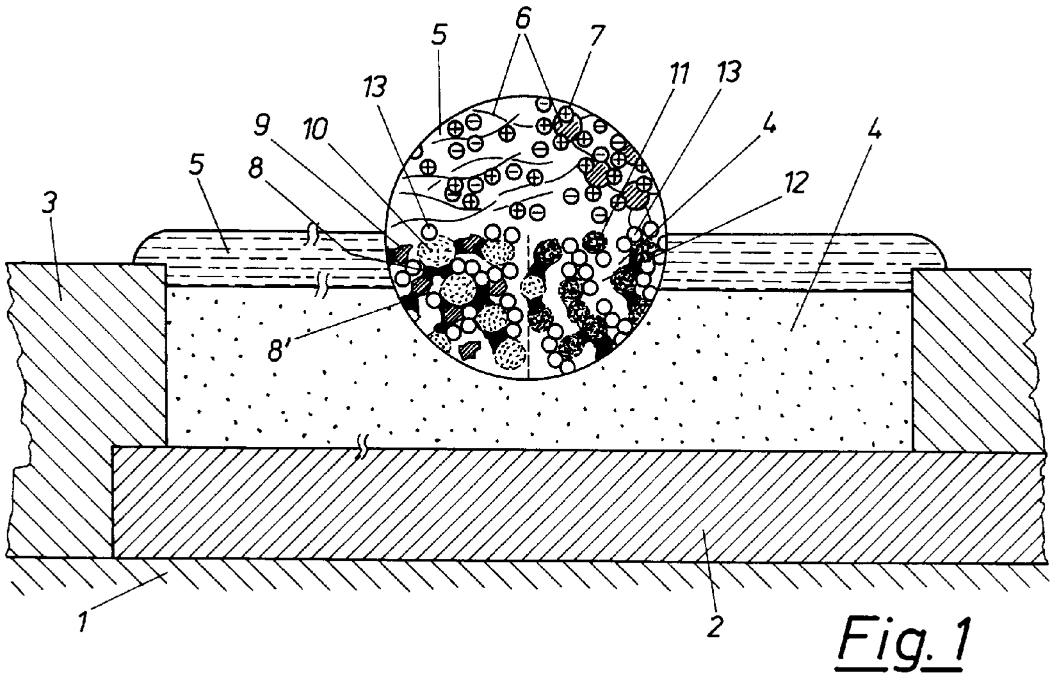


Fig. 1

EP 0 711 837 A2

**Beschreibung**

Die Erfindung betrifft eine amperometrische Enzymelektrode zur Messung der Konzentration eines Enzymsubstrates in einer Probe mit einem auf bzw. in einem elektrisch leitenden, porösen Elektrodenmaterial immobilisierten bzw. adsorbierten Enzym, wobei das Elektrodenmaterial einen redoxinaktiven Leiter mit einem leitfähigen Pigment und einem selbst nicht leitenden Bindemittel und eine darin fein verteilte katalytische Substanz aufweist.

Die Substratbestimmung durch amperometrischen Biosensoren bzw. Enzymelektroden auf Basis der Wasserstoffperoxidmessung mit immobilisierten Oxidoreduktasen ist Gegenstand einer großen Anzahl von Patenten und Publikationen und spielt in der biochemischen Sensorik eine nicht unerhebliche Rolle. Bei der Messung von Substraten wie z.B. Glucose in Körperflüssigkeiten (Blut, Serum, interstitielle Flüssigkeit und Harn) stellt sich das Problem der Interferenzen. So verfälschen viele redoxaktive Metabolite und Medikamente wie z.B. p-Acetamol, Ascorbat, Harnsäure u. a. die Substratbestimmung nach diesem Meßprinzip erheblich bzw. machen diese sogar unmöglich.

In der EP 0 247 850 A1 wird eine Unterdrückung dieser Interferenzen durch den Einsatz von Elektroden erzielt, bei denen fein verteilte Partikel der Platinmetalle auf der Oberfläche einer porösen Schicht bestehend aus Graphit und einem porösen polymeren Bindemittel aufgebracht sind. Durch diese Maßnahme wird die Zersetzungsspannung von $H_2O_2$, die ohne Verwendung von Katalysatoren bei $\geq$ 600 mV liegt, drastisch d.h. unter 400 mV reduziert. Da die Zersetzungsspannung für die redoxaktiven Störsubstanzen nur minimal reduziert wird, kommt es zu einer deutlichen Unterdrückung der Interferenzen.

Die Aufbringung der Platinpartikeln erfolgt in der Regel elektrochemisch durch Redox-Reaktionen bzw. kann durch Besputterung bewerkstelligt werden. Für die Herstellung von Elektroden nach dem Prinzip der Dickschichttechnik bedeuten diese Aufbringungsarten mitunter eine kostenintensive Prozeßmodifizierung.

Wasserstoffperoxid zerfällt autoxidativ in Wasser und Sauerstoff. Bei Raumtemperatur ist die Zerfallsgeschwindigkeit allerdings unmeßbar klein, kann jedoch beispielsweise durch Metalle der Platingruppe katalysiert werden.

Wie aus der EP 0 247 850 A1 hervorgeht, katalisieren die Platinmetalle den autoxidativen Zerfall von $H_2O_2$, wobei bei der für die $H_2O_2$-Bestimmung relevanten Reaktion (1) eine Verschiebung der Zersetzungsspannung $E_o$ von $\geq$ 0.6 V auf < 0.4 V erfolgt.

$$H_2O_2 \longleftrightarrow 2H^+ + 2e^- + O_2 \qquad (1)$$

Durch die hohe Leitfähigkeit der Platinmetallpartikel sind bei diesem Elektrodentyp Katalysator und Ableitelektrode ident.

Aufgabe der vorliegenden Erfindung ist es, ausgehend von eingangs beschriebenen Enzymelektroden eine amperometrische Enzymelektrode vorzuschlagen, welche kostengünstig und einfach herzustellen ist, und auch bei der Herstellung nach dem Prinzip der Dickschichttechnik keine Probleme aufwirft und bei welcher vor allem die Interferenzen redoxaktiver Metabolite weiter unterdrückt werden.

Diese Aufgabe wird erfindungsgemäß dadurch gelöst, daß das Elektrodenmaterial der Enzymelektrode eine probenseitige Deckschicht oder eine in den Poren des Elektrodenmaterials vorliegende Sperrschicht aufweisen, welche aus einem polyionischen Polymer besteht.

Erfindungsgemäß ist weiters vorgesehen, daß das polyionische Polymer aus polykationischen Polymeren, Polykationen bildenden Polymeren oder aus Polymeren besteht, welche sowohl positive als auch negative, kovalent gebundene Gruppen aufweisen, wobei beispielsweise das polykationische Polymer Aminogruppen sowie quarternäre Ammoniumgruppen trägt, oder das sowohl positive als auch negative Gruppen tragende Polymer ein Biopolymer, vorzugsweise ein Protein ist.

Dabei kann z. B. die katalytische Substanz des Elektrodenmaterials auch ein stabiles Oxid oder Oxidhydrat eines Übergangselementes der vierten Periode des Periodensystems der Elemente sein, welches in den redoxinaktiven Leiter eingebettet ist.

Überraschenderweise wurde entdeckt, daß die für den autoxidaven Zerfall relevanten Katalysatoren der genannten Oxide bzw. Oxidhydrate, welche im Vergleich zu den Edelmetallen schlechte Leiter bzw. Isolatoren sind, die Oxidation von $H_20_2$ gemäß obiger Gleichung (1) ebenfalls beeinflussen, d.h. $E_o$ erniedrigen und somit auch zur Interferenzunterdrückung herangezogen werden können.

Zur Herstellung des Elektrodenmaterials kann z.B. $Mn0_2$-Pulver mit Graphit bzw. mit Aktivkohle und Graphit sowie mit einem Polymerbindemittel zu einer Paste verarbeitet, deren Film nach Abdunsten des Lösungsmittels eine mehr oder weniger poröse Struktur und somit eine große aktive Oberfläche besitzt. Ferner kann z.B., $Mn0_2$ durch geeignete Redoxreaktionen auf Aktivkohle gefällt und das resultierende Pulver analog des reinen Manganoxids mit Graphit und Polymerbindemittel in eine siebdruckfähige Paste verarbeitet werden.

Die Herstellung einer erfindungsgemäßen Enzymelektrode wird unter der Verwendung von Braunstein (Mangan (IV)oxid) als Elektrokatalysator näher beschrieben. Die nachfolgend beschriebene Bestimmung von $H_20_2$ neben den

oben angeführten Störsubstanzen wurde bei physiologischen pH-Werten durchgeführt.

**BEISPIELE (Herstellung des Elektrodenmaterials)**

1) 20 Gewichtsteile Mangan(IV)oxid gepulvert werden mit 100 Gewichtsteilen Graphitpaste (z.B. Elektrodag 4233 SS der Firma AchesonColloiden B.V. Scheenda/Niederlande) vermengt und in einer Kugelmühle zu einer homogenen Paste aufgeschlossen.

2) 10 Gewichtsteile Mangandioxid sowie 10 Teile Aktivkohle werden analog Beispiel 1 mit 100 Gewichtsteilen Graphitpaste vermischt.

3a) Fällung von Mangan(IV)oxid auf Aktivkohle:
10 g Aktivkohle werden in 100 ml 1n.NaOH suspendiert und kurzzeitig bis zum Sieden erhitzt. Nach dem Abkühlen wird die Suspension in eine Lösung bestehend aus 1 g $KMn0_2$, und 500 ml 1n.NaOH gegossen. Unter Rühren wird solange Natriumsulfit gelöst in Wasser zugesetzt, bis sich die Lösung entfärbt hat.

3b) Fällung von $Mn0_2$ durch Synproportionierung gemäß Gleichung (2).

$$2\ Mn0_4^- + 3Mn^{3+} + 4\ OH^- \rightarrow 5\ Mn0_2 + 2\ H_20 \qquad (2)$$

10 g Aktivkohle werden in 200 ml 1n.NaOH suspendiert und kurzzeitig zum Sieden erhitzt. Nach Abkühlen wird die Suspension in eine Lösung bestehend aus 0.2 g $KMn0_4$ und 200 ml Wasser eingetragen und mit einer verdünnten Mangan(II)-Lösung solange versetzt, bis Entfärbung eintritt.

Die gemäß Beispiel 3a) und 3b) erzeugten Mn02-Aktivkohle-Gemische werden abfiltriert, mit dest. Wasser gründlich gewaschen und bei 100°C getrocknet.

10 Teile dieser Fällungen werden mit je 50 Gewichtsteilen Graphitpaste verrührt und in einer Kugelmühle analog Beispiel 1) verarbeitet.

Elektrodenpräparationen

Die vorliegenden Elektrolytmaterialien werden jeweils in Form eines 1.5 x 8 mm bzw. 2 x 1 mm großen rechteckigen Feldes auf eine mit Silberleitbahnen bedruckte Plexiglasplatte via Siebdruckverfahren aufgebracht, und die blanken Oberflächen der Silberleitbahnen mit einem Isolierlack abgedeckt.

Zum Zwecke der Interferenzstudien wurden Elektrodenflächen, die aus der Paste nach Beispiel 1) gefertigt wurden, beispielsweise mit polyionischen Polymeren wie Nafion, Albumin oder Polyethylenimin/Albumin im Verhältnis 2:1 beschichtet und die albuminhältigen Beschichtungen nachträglich mit Glutardialdehyd vernetzt.

Elektroden, die entsprechend Beispiel 3a) bzw. 3b) gefertigt wurden, wurden sowohl direkt als auch nach der Beschichtung mit perfluoriertem Nafion (Firma Aldrich-Chemie, Steinheim/BRD), mit 0.2 µl einer 5%igen Glucoseoxidase(GOD)Lösung beschichtet und durch Vernetzung im Glutardialdehyddampf (30 Minuten bei Raumtemperatur) immobilisiert.

Messungen:

Die Messung der einzelnen Parameter erfolgt potentiostatisch als Gegenelektrode (Kathode) fungiert eine Ag/AgCl-Elektrode.

Durch Variation der angelegten Spannung wurden die für die jeweilige Elektrode optimalen Bedingungen eruiert.

Als Referenzsysteme wurden Pt-Blech, Edelmetall-Aktivkohle-Graphitelektroden sowie eine reine Graphitelektrode vermessen.

Eine Gold-Kohleelektrode wurde durch Mischen von Aktivkohle, welche 5% kolloidal verteiltes, durch einen Redoxprozeß aus Goldchlorid abgeschiedenes Gold enthält, analog Beispiel 1 hergestellt. Eine Ruthenium-Kohleelektrode wurde analog präpariert, das Ruthenium wurde jedoch via Ruthenium 5% auf Aktivkohle in die Elektrodenmasse eingebracht. Platinisierter Kohlenstoff wurde gemäß GB 21 91 003 B (H.P. Benetto et al.) präpariert und in Form eines 1.5x8mm großen Elektrodenplättchens ausgetestet.

Weiters wurde das Elektrodenmaterial variiert, indem analog Beispiel 1 $Mn0_2$ durch $Fe_20_3$, $Fe_30_4$, Fe00H, $Cr_20_3$ sowie $V_20_5$ ersetzt wurde.

Als Testmedien wurden $H_20_2$ (1.0, 2.0 und 5.0 mM), Ascorbat, p-Acetamol, Urat (jeweils 1.0 mM) sowie Glucose (2.5, 5.0. 10.0 mM), alle gelöst in Standard A (ISE-Elektrolyt der Fa. AVL MEDICAL INSTRUMENTS, pH = 7.38 sowie physiologisch relevanter ionaler Hintergrund) verwendet; als Referenz wurde ebenfalls Standard A eingesetzt.

Gemessen wurde der Strom in Abhängigkeit von den einzelnen Parametern. In Tabelle I werden $Mn0_2$-Elektroden den Edelmetallelektroden (Au/C, Ru/C, Pt-Blech sowie platinisierter Kohlenstoff) sowie einer reinen Graphitelektrode in puncto optimale Spannung, Änderung des Stromflusses I(µA) bei Variation der Konzentration des redoxaktiven Analyten ($H_20_2$) bzw. der Störer gegenübergestellt, sowie die $H_20_2$-Konzentration angegeben, die den Stromfluß bei

Vorliegen von ImM interferierender Substanz entspricht. Bei dieser Messerie weisen die Elektroden keine polyionische Deckschicht auf.

TABELLE I

| | I (µA) bei Wechsel Std.A-Analytlösung | | | | | $H_2O_2$-Konzentration entspricht 1 mM | | | |
|---|---|---|---|---|---|---|---|---|---|
| Elektrodenmat. | $H_2O_2$ 1mM | $H_2O_2$ 5mM | Ascorbat 1mM | p Acetamol 1mM | Urat 1mM | Ascorbat | p Acetamol | Urat | Arbeits-Spannung |
| $MnO_2$/C | | | | | | | | | |
| gemäß 1 | -10.0 | -42.5 | -7,0 | -2,0 | -3,0 | 0.7 | 0.2 | 0.3 | 350 |
| 2 | -11,0 | -47,0 | -7,5 | -1,9 | -2,2 | 0.68 | 0.17 | 0.2 | 350 |
| 3a | -5.5 | -25,0 | -3.2 | -0.9 | -1.3 | 0.58 | 0.16 | 0.23 | 350 |
| 3b | -6.2 | -29.0 | -4.0 | -1.3 | -2.0 | 0.64 | 0.20 | 0.32 | 350 |
| Au/C | -0.65 | -1.65 | -15.7 | -5.8 | -3.4 | 24.2 | 8.90 | 5.23 | 500 |
| Ru/C | -3.0 | -8.6 | -7.0 | -4.4 | -2.0 | 2.33 | 1.46 | 0.66 | 400-500 |
| Pt-Blech | -2,6 | -5.5 | -6.0 | -1.0 | -0.6 | 2.30 | 0.38 | 0.23 | 450-500 |
| platinisierter Kohlenstoff | -6.0 | -32 | -3.0 | 0.0 | -2,5 | 0.5 | 0 | 0.41 | 350 |
| Graphit | -0.1 | -0.25 | -12,0 | -10.0 | -4.0 | 121 | 100 | 40 | 600 |

Die Erfindung wird im folgenden anhand von Zeichnungen näher erläutert. Es zeigen:

Fig. 1 eine schematische Schnittdarstellung einer amperometrischen Enzymelektrode nach der Erfindung,
Fig. 2 und Fig. 3 Details aus Fig. 1,
Fig. 4 eine Anordnung von Enzymelektroden,
Fig. 5 einen Schnitt gemäß Linie V-V in Fig. 4, sowie die
Fig. 6a, 6b, 7a bis 7c und 8 Meßdiagramme mit erfindungsgemäßen Enzymelektroden.

Gemäß Fig. 1 ist auf einem Träger 1 eine metallisch leitende Schicht 2, z.B. eine Silberleitbahn, aufgebracht, auf der sich ein elektrokatalytisches, poröses Elektrodenmaterial 4 befindet, sowie die darüber angeordnete permeationsselektive Deckschicht 5 angedeutet ist. Eine Isolierschicht 3 verhindert einen direkten Kontakt zwischen der Probe und der leitenden Schicht 2.

Das Elektrodenmaterial 4 weist ein poröses Gefüge auf und besteht aus Aktivkohlepartikel 10 und Partikel 9 einer katalytischen Substanz, beispielsweise Mangandioxidpartikel (linke Seite von Fig. 1) bzw. aus Aktivkohlepartikeln 11, an deren Oberfläche die katalytische Substanz feinst verteilt vorliegt (rechte Seite von Fig. 1). Die einzelnen Partikel 9, 10 und 11 werden durch ein Bindemittel 8 verkittet, in welchem sich als leitfähiges Pigment 8' Graphitpulver befindet. Die katalytische Substanz aus einem Oxid oder Oxidhydrat eines Übergangselementes der vierten Periode ist somit in einem redoxinaktiven Leiter aus einem Bindemittel 8 mit einem leitfähigen Pigment 8' eingebettet. In den Poren 12 des Elektrodenmaterials 4 ist das Enzym 13, beispielsweise Glucoseoxidase, adsorbtiv gebunden bzw. durch Vernetzung chemisch fixiert.

Die als permeationsselektive Interferenzsperrschicht fungierende Deckschicht 5 besteht aus einem linearen bzw. vernetzten globulären Polymergerüst 6, an dem positiv geladene Gruppen kovalent gebunden vorliegen. Aufgrund der Elektroneutralitätsbeziehung sind diese gebundenen Kationen mittels austauschbaren kleinen negativen Gegenionen (elektrostatisch fixierte Anionen 7') ladungskompensiert.

Die in den Poren 12 des porösen Elektrodenmaterials 4 ablaufenden chemischen bzw. elektrochemischen Reaktionen sind schematisch in den Fig. 2 und 3 dargestellt.

In Fig. 2 werden ein $MnO_2$-Partikel 9 über Graphitpartikel 8' kontaktiert, welche mit einem Polymerbindemittel 8 zusammengekittet sind. In Fig. 3 sind die $MnO_2$-Partikel 9 fein verteilt an die Aktivkohlepartikel 10 angelagert. Mit 13 ist das Enzym, beispielsweise Glucoseoxidase, gekennzeichnet.

Die Fig. 4 und 5 zeigen eine Elektrodenanordnung, bei welcher auf einem Träger 1, beispielsweise einer Plexigasplatte mit Silberleitbahnen 2, das Elektrodenmaterial 4 in Form von 1,5 x 8 mm bzw. 2 x 1 mm großen rechteckigen Feldern aufgebracht wird. Die blanken Oberflächen der Silberleitbahnen können mit einer Isolierschicht 3 abgedeckt sein.

Tabelle II zeigt den Einfluß polyionischer Elektrodenbeschichtungen auf die Interferenzunterdrückung an einer $Mn0_2$-Graphitelektrode.

TABELLE II

| $C_{H202}$ (mM) entspricht 1mM | | | | OPTIMALE ARBEITS-SPANNUNG (mV) |
|---|---|---|---|---|
| ELEKTRODE | ASCORBAT | P ACETAMOL | URAT | |
| $MnO_2$/C gemäß Beispiel 1 | 0.7 | 0.2 | 0.3 | 350 |
| +Nafion perfluoriert (Aldrich) | 0.09 | 0.07 | 0.21 | 340 - 350 |
| +Polyethylenimin/Albumin 2:1-m-Glu-tardialdehyd 3D-vernetzt | 0.21 | 0.08 | 0.07 | 350 |
| +Protein (Rinderserumalbumin mit Glutaldehyd 3 DI vernetzt | 0.1 | 0.15 | 0.05 | 350 |

Die Fig. 6a und 6b zeigen den Stromfluß I(µA) versus Konzentration C(mM) der redoxaktiven Parameter (o = $H_2O_2$, x = p.Acetatamol, + = Ascorbat = Urat) gemessen an einer $Mn0_2$-Graphitelektrode (a) sowie an einer aus platinisiertem Kohlenstoff präparierten Elektrode (b).

In den Fig. 7a bis 7c ist der Stromfluß I(µA) versus Konzentration C(mM) der redoxaktiven Parameter (gleiche Parameter wie Fig. 6a, 6b) gemessen an einer Mn02-Graphitelektrode (a und b) sowie an platinisierten Kohlenstoff (c) aufgetragen. Die Elektroden sind wie folgt modifiziert:

a = $Mn0_2$/C-Nafion-GOD

b = $MnO_2$/C-GOD
c = platinisierter Kohlenstoff-Nafion-GOD

Fig. 8 zeigt den Stromfluß I(μA) bei einer Spannung von 350 mV versus Glucosekonzentration bei einer optimierten erfindungsgemäßen Elektrode, bei der die permselektive Schicht auf die das Enzym beinhaltende Elektrodenschicht gemäß Fig. 1 aufgebracht ist. Die Funktion Strom versus Glucosekonzentration ist im Bereich von 0 bis 250 mg/dl (12,5 mM) linear.

Erfindungsgemäß kann das polyionisierte Polymer auch als Bindemittel für die pulver- bzw. partikelförmigen Elektrodenkomponenten fungieren.

Schließlich kann das polyionische Polymer das Enzym immobilisiert oder adsorbiert enthalten.

Die Messungen mit den erfindungsgemäßen Elektroden haben gezeigt, daß $MnO_2$-Graphit-Elektroden in puncto Verschiebung der Zersetzungspannung ein dem platinisierten Kohlenstoff analoges Verhalten zeigen, d.h. in Bezug auf Elektrokatalyse ebenbürtig sind. Erstaunlich ist, daß eine derartige starke Verschiebung weder bei Platinblech noch bei Ruthenium- bzw. Gold-Aktivkohle-Graphitelektroden beobachtet werden kann.

Bei den in der Tabelle I nicht ausgewiesenen Eisen-, Chrom- und Vanadiumoxid-Graphitelektroden (Herstellung analog Beispiel 1 der $MnO_2$-Kohleelektrode) konnte ein im Vergleich zu $MnO_2$ wesentlich schwächere Verschiebung der Zersetzungsspannung beobachtet werden, wobei folgende Reihenfolge auffiel: $MnO_2$ > FeOOH > $Fe_3O_4$ > $Fe_2O_3$-gebrannt > $Cr_2O_3$, > $Fe_2O_3$-mineralisch (Hämatit).

Wie aus der Tabelle I ebenfalls ersichtlich ist, nimmt mit der Verschiebung der Zersetzungsspannung auch die Interferenzunterdrückung zu.

Bezüglich der Aufbringung bzw. Einbringung polyionischer Polymere hat sich ergeben, daß die anionischen Interferenten (Ascorbat, Urat sowie der dissoziierte Anteil von p. Acetamol) z.B. durch Beziehen der Elektrode mit einer polyanionischen Deckschicht durch elektrostatische Abstoßung an der Wanderung zur Anode gehindert werden.

Aus Tabelle II ist ersichtlich, daß dieser Effekt nicht nur durch Polymere mit negativ geladenen Gruppen sondern auch durch überwiegend positiv geladene Polymerme aber auch durch generell polyionische Polymere erzielt werden kann. In diesem Fall scheint weniger die elektrostatische Barriere an der Grenzfläche zwischen Polymer und Probenmedium sondern vielmehr der Ionentauscheffekt solcher Polymere (Absenken der Diffusionsrate in Polymerfilm durch permanentes Ad- bzw. Desorbieren der Störionen beim Durchtritt) analog der Ionentausch-Chromatographie eine Rolle zu spielen. Im Falle von Proteinsperrschichten tritt somit bei den unter physiologischen Bedingungen vollständig dissoziierten Störern eine deutliche Interferenzunterdrückung auf.

Tabelle III sowie die Fig. 7a-7c und 8 zeigen, daß im Bezug auf die $H_2O_2$ und Glucose-Messung bei der erfindungsgemäßen Elektrode im Vergleich zur Edelmetallkatalyse eine zum Teil bessere Interferenzunterdrückung gewährleistet ist.

TABELLE III

| | mM Glucose entspricht 1mM | | |
|---|---|---|---|
| ELEKTRODE | ASCORBAT | P ACETAMOL | URAT |
| $MnO_2$/C + Glucoseoxidase immobilisiert | + 1 | + 0.7 | + 0.5 |
| $MnO_2$/C + Nafion + Glucoseoxidase immobilisiert | - 0.2 | + 0.2 | 0 |
| Platinisierter Kohlenstoff + Nafion + Glucoseoxidase immobilisiert | 0 | + 5 | - 4 |

## Patentansprüche

1. Amperometrische Enzymelektrode zur Messung der Konzentration eines Enzymsubstrates in einer Probe mit einem auf bzw. in einem elektrisch leitenden, porösen Elektrodenmaterial (4) immobilisierten bzw. adsorbierten Enzym (13), wobei das Elektrodenmaterial (4) einen redoxinaktiven Leiter mit einem leitfähigen Pigment (8') und einem selbst nicht leitenden Bindemittel (8) und eine darin fein verteilte katalytische Substanz (9) aufweist, **dadurch gekennzeichnet**, daß das Elektrodenmaterial (4) eine probenseitige Deckschicht (5) oder eine in den Poren (12) des Elektrodenmaterials (4) vorliegende Sperrschicht aufweist, welche aus einem polyionischen Polymer besteht.

2. Enzymelektrode nach Anspruch 1, **dadurch gekennzeichnet**, daß das polyionische Polymer aus polykationischen Polymeren, Polykationen bildenden Polymeren oder aus Polymeren besteht, welche sowohl positive als auch negative, kovalent gebundene Gruppen aufweisen.

3. Enzymelektrode nach Anspruch 2, **dadurch gekennzeichnet**, daß das polykationische Polymer Aminogruppen sowie quarternäre Ammoniumgruppen trägt.

4. Enzymelektrode nach Anspruch 2, **dadurch gekennzeichnet**, daß das sowohl positive als auch negative Gruppen tragende Polymer ein Biopolymer, vorzugsweise ein Protein ist.

5. Enzymelektrode nach einem der Ansprüche 2 bis 4, **dadurch gekennzeichnet**, daß das polyionische Polymer als Bindemittel (8) für die pulver- bzw. partikelförmigen Elektrodenkomponenten (8', 9, 10) fungiert.

6. Enzymelektrode nach einem der Ansprüche 2 bis 5, **dadurch gekennzeichnet**, daß das polyionische Polymer das Enzym (13) immobilisiert oder adsorbiert enthält.

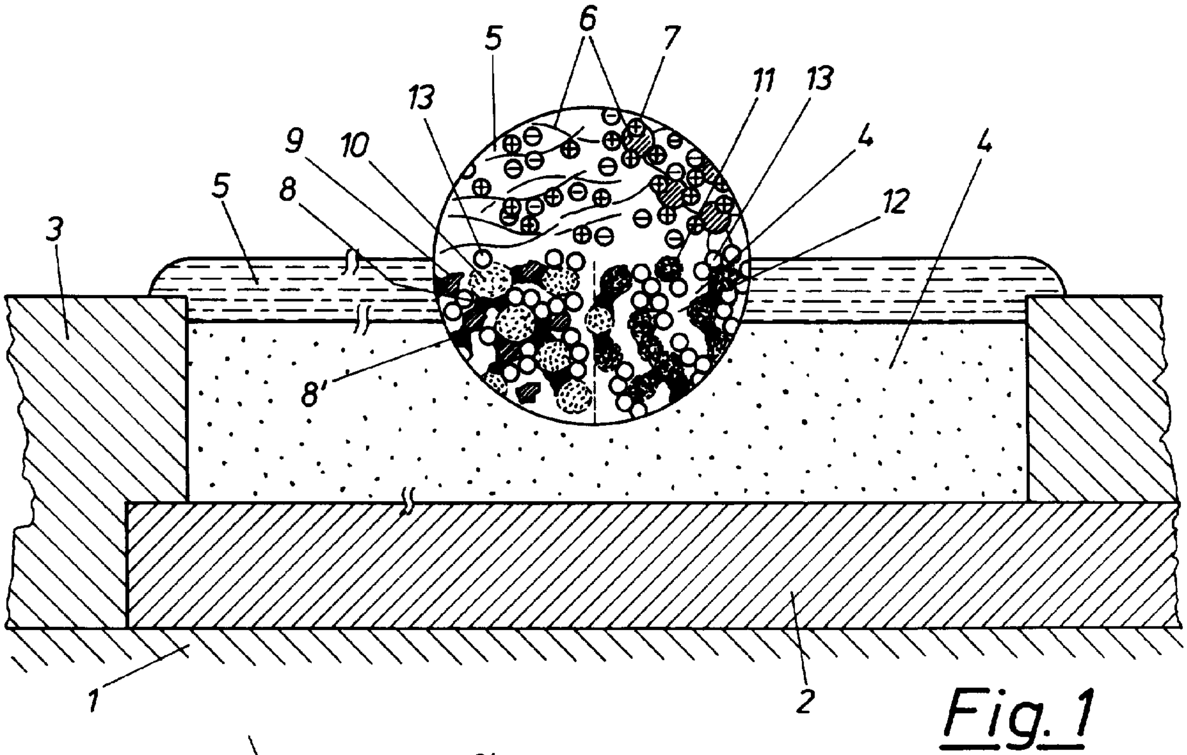


Fig. 1

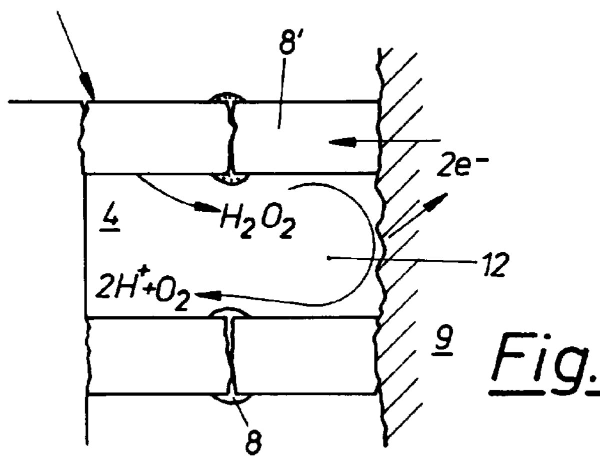


Fig. 2

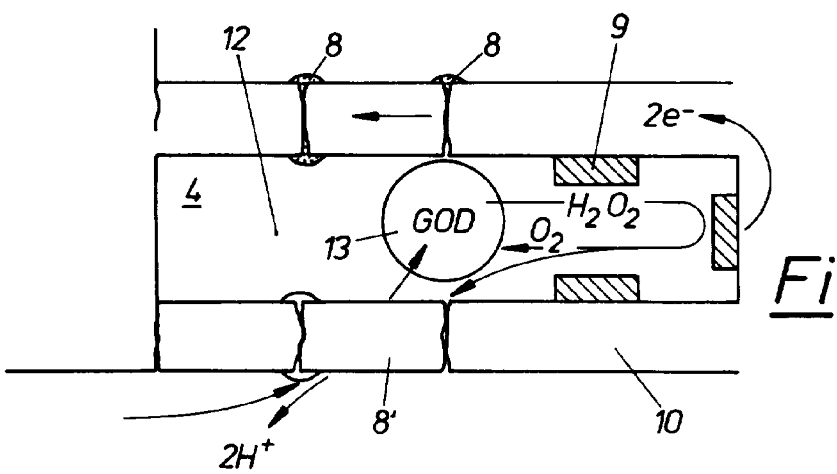


Fig. 3

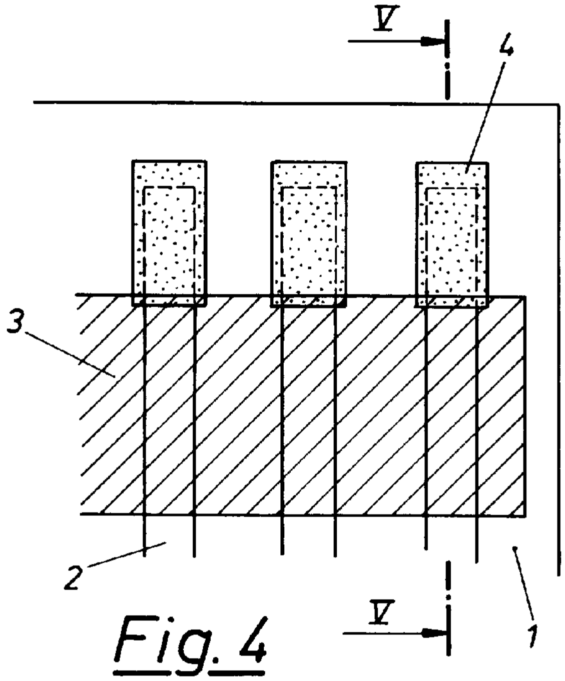


Fig. 4

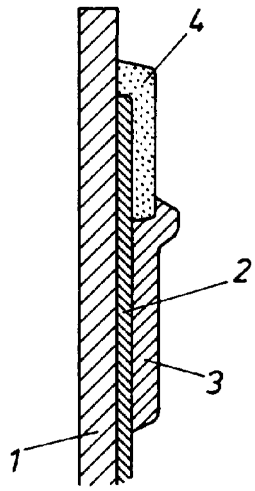


Fig. 5

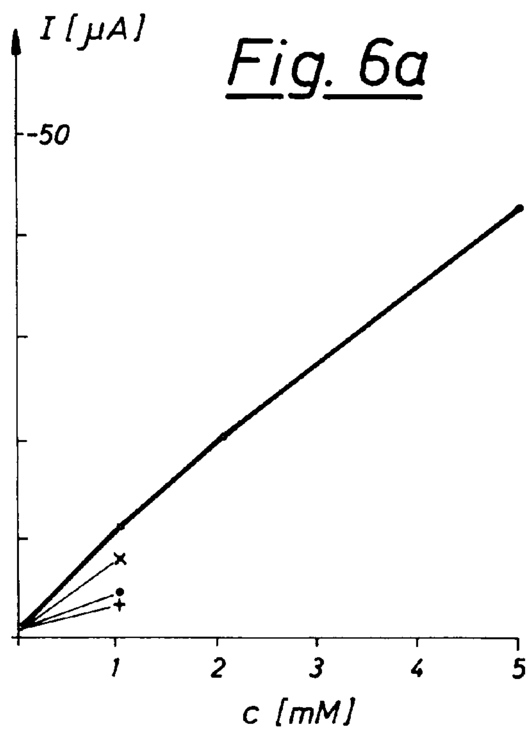


Fig. 6a

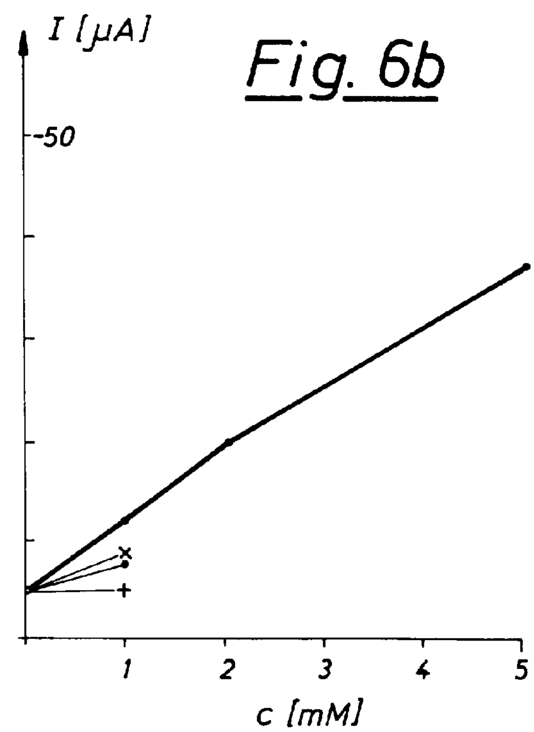


Fig. 6b

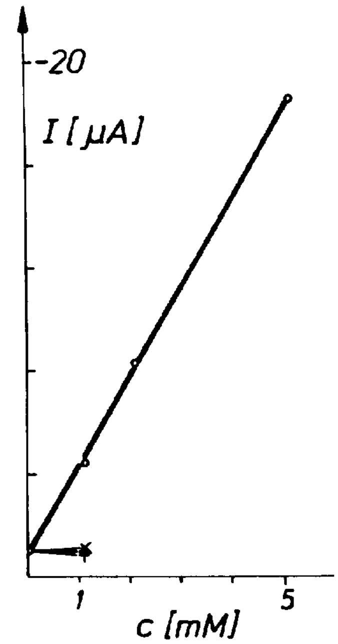


Fig. 7a

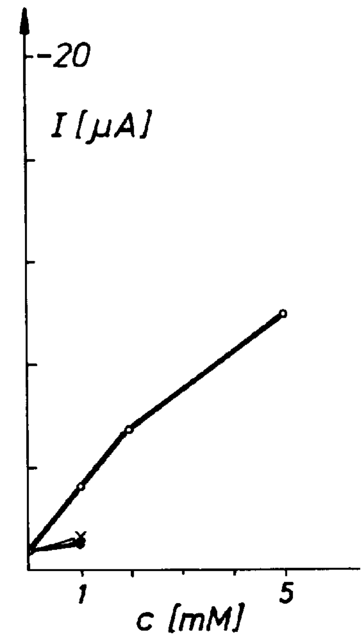


Fig. 7b

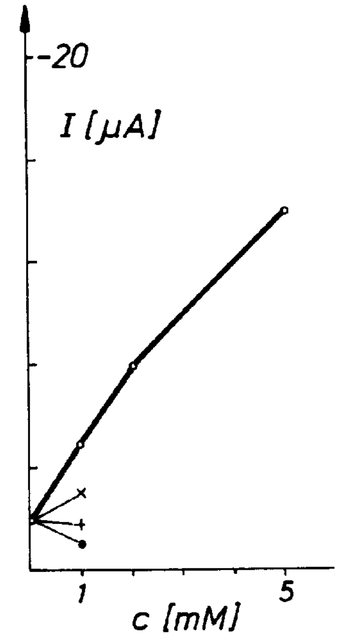


Fig. 7c

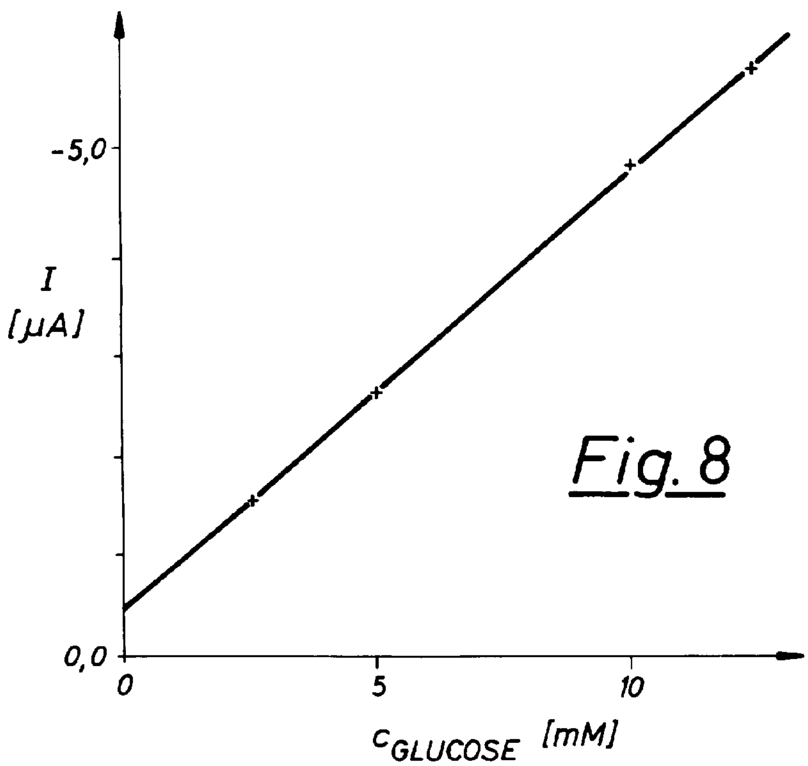


Fig. 8